# EUROPÄISCHE PATENTANMELDUNG

(11) **EP 2 033 657 A1**
(43) Veröffentlichungstag der Anmeldung: **11.03.2009**
(21) Anmeldenummer: 07115602.0
(22) Anmeldetag: 04.09.2007
(51) Int. Cl.: A61K 39/395, A61P 35/00, C07K 16/30, C07K 16/32, C07K 16/28

(54) **Intraoperative trifunktionale Antikörper-Applikation zur Prophylaxe intraperitonealer Tumorzelldissemination**

(71) Anmelder: TRION PHARMA GMBH, 80807 München (DE)
(72) Erfinder: Lindhofer, Horst, 80639 München (DE); Heiss, Markus M., Dr., 50968 Köln (DE)
(74) Vertreter: Behnisch, Werner

(57) **Zusammenfassung**

Die Erfindung beschreibt die Verwendung trifunktioneller bispezifischer und trispezifischer Antikörper zur Zerstörung von Tumorzellen, die z. B. durch einen chirurgischen Eingriff intraperitoneal disseminiert werden, wobei der Antikörper intraoperativ, bevorzugt direkt lokal in die Bauchhöhle, verabreicht wird.

## Beschreibung

Die Erfindung betrifft die Verwendung eines trifunktionellen bispezifischen und/oder trispezifischen Antikörpers zur Herstellung einer pharmazeutischen Zusammensetzung zur Zerstörung von z. B. durch einen chirurgischen Eingriff intraperitoneal disseminierten Tumorzellen.

Ein bislang ungelöstes Problem in der chirurgischen Onkologie stellt die intraoperative Tumorzelldissemination dar. Es ist akzeptiert, dass es bei den meisten intraabdominellen Tumoroperationen durch den operativen Eingriff zur Ablösung und Dissemination von Tumorzellen kommt, die sich im portalvenösen Blut, im peripheren Blut, im Knochenmark, im Wundblut, aber vor allem innerhalb der Peritonealhöhle verteilen. Tumorbiologisch ist die Dissemination von Zellen in die systemische Blutzirkulation anders zu werten als die Dissemination in die freie Bauchhöhle. Der Nachweis von Tumoreinzelzellen im Blut wurde bereits in den 50er- und 60er Jahren geführt, ohne allerdings einen klaren Bezug zur Prognose darstellen zu können.

Auch mit modernen immunozytochemischen und molekularbiologischen Methoden ist es bislang nicht gelungen, auf überzeugendem Evidenzniveau den Nachweis von systemisch disseminierten Tumoreinzelzellen als Indikatoren einer minimal-residualen Tumorerkrankung mit der tatsächlichen klinischen Prognose des Patienten eindeutig zu korrelieren und dies als Prognose klar zu definieren.

Die Situation ändert sich aber, wenn unterschiedliche Körperkompartimente wie beispielsweise das Knochenmark betrachtet werden. Es gibt Evidenz dafür, dass sich Tumorzellen, die im Knochenmark nachgewiesen worden sind, bereits intestitiell befinden und damit bereits die ersten Schritte in der Extravasation und Invasion von mesenchymaler Matrix erfolgreich absolviert haben. Zumindest für das Mammakarzinom konnte eine Prognoseassoziation zwischen Tumorzellennachweis im Kompartiment Knochenmark und im Auftreten von Skelettmetastasen, aber auch in der Langzeitprognose nachgewiesen werden.

Anders verhält es sich, wenn einzelne Tumorzellen in der freien Bauchhöhle intraperitoneal gefunden werden. Ein zytologischer oder auch immunzytologischer Nachweis von Tumorzellen intraperitoneal bei Tumoren des Gastrointestinaltrakts, wie dem Magenkarzinom und dem kolorektalen Karzinom, sind eindeutige Risikoindikatoren für das Auftreten einer Peritonealkarzinose. Diese intraperitoneale Tumordissemination tritt umso häufiger auf, je ausgeprägter die Infiltrationstiefe des Karzinoms insbesondere in die Serosa ist. Bei makroskopischer Diagnose eines Serosabefalls fanden sich in begleitenden zytologischen Untersuchen mit sehr hoher Wahrscheinlichkeit positive Tumorzellnachweise.

Bei der Tumoroperation kommt nun erschwerend hinzu, dass durch die Präparation der Gewebe und die Manipulation des tumortragenden Organs eine zusätzliche Tumordissemination auftritt. Hier ergibt sich die Fragestellung, wie das Risiko einer solchen Tumordissemination intraoperativ beseitigt werden kann. Bislang waren entsprechende Versuche mit einer intraoperativen Chemoperfusion weniger erfolgreich und verbieten sich auch aufgrund der Komplexität des Verfahrens und des Risikos für eine breite Anwendung. Auch hat sie den Nachteil, nur proliferierende Tumorzellen erreichen zu können.

Es ist daher eine Aufgabe der Erfindung, eine pharmazeutische Zusammensetzung bereitzustellen, die zur Zerstörung von Tumorzellen geeignet ist, welche sich entweder vom Tumor, z. B. vom Primärtumor oder seinen Metastasen, ohne Fremdeinwirkung, z. B. durch einen chirurgischen Eingriff, losgelöst, d. h. disseminiert haben und in der Bauchhöhle bzw. im Bauchraum verteilt vorliegen, oder die durch einen chirurgischen Eingriff zur Tumorresektion im Bauchraum bzw. in der Bauchhöhle disseminiert werden, um hierdurch das Rezidivrisiko zu vermindern.

Diese Aufgabe wird erfindungsgemäß durch die Herstellung einer pharmazeutischen Zusammensetzung gelöst, die, neben üblichen Träger- und/oder Hilfsstoffen, einen trifunktionellen bispezifischen und/oder trispezifischen Antikörper mit den nachfolgenden Eigenschaften enthält:
(a) bindet an eine T-Zelle;
(b) bindet an zumindest ein tumorassoziiertes Antigen, welches auf intraperitoneal vorkommenden Tumorzellen exprimiert wird;
(c) bindet durch seinen Fc-Teil (bei trifunktionalen bispezifischen Antikörpern) oder durch eine dritte Spezifität (bei trispezifischen Antikörpern) an Fc-Rezeptor positive Zellen.. Bevorzugte Ausgestaltungen dieser Antikörper werden nachfolgend näher beschrieben. Die nachfolgend näher beschriebene intraoperative Applikation.ist integraler Bestandteil der erfindungsgemäßen Problemlösung ist

Diese Zusammensetzung wird erfindungsgemäß intraoperativ appliziert. In einer besonders bevorzugten Ausführungsform erfolgt die Applikation lokal direkt in die Bauchhöhle, d. h. in den Bauchraum. Hierdurch wird eine Zerstörung von z. B. durch den chirurgischen Eingriff bei der Resektion des Primär- und/oder Sekundärtumors disseminierten Tumorzellen erreicht, die ansonsten über die Körperflüssigkeiten in den Körper des Patienten verteilt und die Wahrscheinlichkeit eines erneuten Tumorwachstums, d. h. von Tumormetastasen deutlich erhöhen würden. Die gleiche therapeutische bzw. prophylaktische Anwendung erfolgt zur Zerstörung von Tumorzellen, welche sich vom Primär- oder Sekundärtumor ohne chirurgischen Eingriff losgelöst haben, das heißt die erfindungsgemäß eingesetzten trifunktionellen und/oder trispezifischen Antikörper werden intraoperativ, in einer bevorzugten Ausgestaltung der Erfindung direkt lokal in die Bauchhöhle, appliziert, und zwar in Verbindung mit einem chirurgischen Eingriff, welcher der Entfernung des Primär- und/oder Sekundärtumors dient.

Die erfindungsgemäße Applikation zur Zerstörung von z. B. durch einen chirurgischen Eingriff intraperitoneal disseminierten Tumorzellen und zur Prophylaxe und Therapie von Tumorrezidiven, die durch intraperitoneal disseminierte Tumorzellen verursacht werden, unabhängig davon, ob diese durch einen chirurgischen Eingriff verursacht wurden, erfolgt aber immer in Verbindung mit einer Applikation der Antikörper während einer Operation, die zur Entfernung des Primärtumors oder von Tumormetastasen erfolgt.

Diese Applikationsart direkt nach einer Tumorresektion, das heißt intraoperativ, beborzugt direkt und lokal, hat den großen Vorteil, dass die Zielzellen der trifunktionellen bispezifischen bzw. trispezifischen Antikörper, nämlich die frei disseminierten Tumorzellen im Bauchraum, gut erreicht werden können. Es ist bekannt, dass disseminierte Tumorzellen sich bereits innerhalb von 12-24 Stunden an die Peritonialoberfläche ansiedeln und ein erstes primitives Tumorstroma aus Fibrin und interzellulärer Matrix aufbauen. Ein weiterer Vorteil der Anwendung der erfindungsgemäß eingesetzten trifunktionellen bispezifischen und/oder trispezifischen Antikörper liegt in der Prophylaxe von z. B. durch den chirurgischen Eingriff intraperitoneal disseminierten Tumorzellen verursachter Tumorrezidive.

Es ist deshalb notwendig, diese Tumorzellen vor diesem Zeitraum von 12-24 Stunden durch die pharmazeutische Zusammensetzung zu erreichen, da durch die Wiederansiedlung der Tumorzellen und den Aufbau der Tumorstroma ihre Erreichbarkeit für die Antikörper schwieriger wird. Die erfindungsgemäß gefundene Lösung, die hier beschriebenen trifunktionellen bispezifischen und/oder trispezifischen Antikörper intraoperativ nach Tumorresektion, bevorzugt direkt lokal in der Bauchhöhle, einzusetzen, bildet den entscheidend neuen Ansatz zu einer erfolgreichen perioperativen Tumortherapie, insbesondere zur Prophylaxe von Tumorrezidiven, insbesondere peritonealen Karzinomatosen, durch Zerstörung frei disseminierter Tumorzellen in der Bauchhöhle.

Bei der peritonealen Karzinomatose handelt es sich um eine Tumorerkrankung, bei der üblicherweise konventionelle Therapieformen wie Chemotherapie, Radiotherapie oder chirurgische Verfahren zu keinen befriedigenden klinischen Ergebnissen führten. Die Prognose für Patienten mit einer peritonealen Karzinomatose ist deshalb auch äußerst ungünstig mit mittleren Überlebensraten von 3-6 Monaten, je nach Art des Primärtumors. Eine der Hauptfaktoren bei der Entwicklung der peritonealen Karzinomatose ist die oben beschriebene intraperitoneale Dissemination von Tumorzellen, die insbesondere durch die chirurgische Behandlung des Tumors, also eine Tumorresektion, verursacht wird, aber auch durch eine Penetration des Tumors oder durch eine seröse Infiltration des Tumors. Persistierende intraperitoneale Tumorzellen, die in das Peritoneum durch Adhäsionsmoleküle und Fibrinschichten eingebettet sind, können sich zu einer makroskopisch sichtbaren Peritonealkarzinomatose entwickeln. Die zur Zeit verfügbaren Untersuchungen zeigen, dass die Persistenz intraabdominaler Tumorzellen bei 100% der betroffenen Patienten zu einer Entwicklung einer peritonealen Karzinomatose führen. Dies ist von besonderem Interesse bei Patienten mit einer chirurgischen Resektion des Primärtumors oder eines Tumorrezidivs und führt zu einem erhöhten Risiko für das Auftreten intraabdominaler Tumorzellen. Die einzige Möglichkeit, eine peritoneale Karzinomatose zu verhindern, liegt in der Zerstörung persistierender intraabdominaler Tumorzellen. Erfindungsgemäß wurde überraschend gefunden, dass die hier beschriebenen, an sich bekannten trifunktionellen bispezifischen und trispezifischen Antikörper bei einer intraoperativen Applikation direkt nach Resektion des Tumors disseminierte Tumorzellen effizient zerstören und die Entwicklung einer peritonealen Karzinomatose verhindern können.

Ein weiterer Grund für den erfindungsgemäß vorgeschlagenen Weg einer intraoperativen Verabreichung des trifunktionellen Antikörpers liegt in der postoperativen Bildung intraabdominaler Fibrinplaques und -adhäsionen, welche die disseminierten Tumorzellen bedecken und damit die Wirksamkeit einer Antikörperbehandlung behindern würden. Eine intraoperative Gabe der Antikörper, bevorzugt in Verbindung mit einer frühzeitigen zusätzlichen Verabreichung des trifunktionellen Antikörpers nach der Resektion, ermöglicht eine optimale Verteilung des Antikörpers in der Peritonealhöhle.

Die erfindungsgemäß eingesetzten trifunktionellen bispezifischen und trispezifischen Antikörper sind an sich bekannt und werden nachfolgend näher beschrieben. Diese trifunktionellen Antikörper sind auch bereits beschrieben worden, um sie zur Zerstörung von Tumorzellen einzusetzen. Neu und überraschend ist jedoch, dass diese Antikörper auch erfolgreich während eines chirurgischen Eingriffs nach der Tumorresektion in der Bauchhöhle, bevorzugt direkt am Operationsort, d. h. lokal in die Bauchhöhle appliziert werden, um dort intraperitoneal disseminierte Tumorzellen, die beispielsweise durch den Eingriff vom Tumorkörper losgelöst wurden oder bereits dort durch Dissemination vom Tumor vorlagen, zu zerstören und somit ihre Ausbreitung im Körper und das Entstehen von Tumorrezidiven zu verhindern. Erfindungsgemäß werden also die trifunktionellen Antikörper zur Prophylaxe und Therapie von Tumorrezidiven eingesetzt, die insbesondere aufgrund des chirurgischen Eingriffs und der hierdurch disseminierten Tumorzellen verursacht werden, und zwar während der Operationsphase, solange die Bauchhöhle geöffnet und noch nicht verschlossen ist. Die erfindungsgemäß beschriebene Applikationsart kann aber auch erfolgreich zur Zerstörung von Tumorzellen eingesetzt werden, die sich "selbstständig" ohne Einwirkung von außen vom Tumor losgelöst haben und die sich in der Bauchhöhle befinden. In einer Ausgestaltungsform der Erfindung werden die Antikörper nicht direkt lokal, sondern systemisch zugeführt; entscheidend aber ist immer, dass die Applikation während der Operationsphase, also intraoperativ erfolgt, also in einer Phase, während der sich der Patient in einer immunsuppressiven Situation befindet. Die Verabreichung von Antikörpern während der Operation war bisher aus den hier beschriebenen Gründen einer Immunsuppression kontraindiziert.

Gemäß dem Stand der Technik war es nicht üblich, intraoperativ, z. B. direkt lokal in die Bauchhöhle, Antikörper zu applizieren, um Tumorzellen zu zerstören. Der Grund liegt in einer mit dem chirurgischen Eingriff verbundenen immunsuppressiven Situation, die nachfolgend näher beschrieben wird.

Durch die bei einer Operation verursachte lokale Gewebeschädigung wird eine akute Entzündungsreaktion ausgelöst, welche die Aktivierung einer Reihe von Protein-Kaskaden, wie z. B. dem Komplementsystem, der Koagulation, der Fibrinolyse und dem Kinin-Kallikrein-System beinhaltet. Es kommt zur Vasodilatation, gesteigerten Kapillarpermeabilität und zum Verlust von Immunglobolinen, Komplement und anderen Proteinen in das Gewebe. Außerdem wird eine Reihe lokaler Mediatoren freigesetzt, z. B. Cytokine und Eicosanoide, die verschiedene hormonell gesteuerte Prozesse beeinflussen. Man vermutet, dass sich diese Stressantwort bzw. die mit ihr verbundenen Komplikationen gerade bei Hochrisikopatienten wie Tumorpatienten auf die Überlebensrate der Patienten negativ auswirken, in dem sie z. B. das Immunsystem stören. Postoperative Infektionen und ein erhöhtes Metastasenrisiko durch perioperativ verschleppte Tumorzellen, also der intraperitoneal disseminierten Tumorzellen, wirken sich auf die postoperative Morbidität und Mortalität aus.

Die Eröffnung der Bauchhöhle, d. h. die Laparotomie mit nachfolgender Tumorresektion durch übliche operative Techniken, führen zu einer endokrinen Stressantwort und einer perioperativen Immunsuppression. Typisch für Operationen ist eine Leukozytose mit gesteigertem Neutrophilen- und Monocytenanteil und erniedrigtem Eosinophilen- und Lymphozytenanteil. Die Akute-Phase-Proteine sind erhöht, die Komplement-Komponenten erniedrigt und deren Spaltprodukte wiederum erhöht.

Bei der zellvermittelten Immunität erniedrigt sich vor allem nach großen Eingriffen wie einer Laparotomie die absolute Anzahl der Lymphozyten und erreicht erst in einigen Tagen postoperativ erneut ihre Ausgangswerte. Die Anzahl der B- und T-Lymphozyten sinkt, und ihr Verhältnis ändert sich zugunsten der B-Zellen. Eine verringerte T-Zell-Proliferation, begleitet von verschiedenen Lymphokinveränderungen, führt, wie oben beschrieben, zu einem erhöhten Infektionsrisiko, einer erhöhten Mortalitätsrate usw.

In der humoralen Immunität sinkt auch die Anzahl der B-Lymphozyten, ihre Proliferationsantwort ist unterdrückt.

In zahlreichen Studien hat sich eine Depression der zellvermittelten Immunität in der peri-und postoperativen Phase gefunden, verbunden mit einer Reduktion der Zellzahl zirkulierender Lymphozyten und Natürlicher Killerzellen, Beeinträchtigung der Aktivität natürlicher Killerzellen, Depression der T-Zell-Proliferation und verminderter Neutrophilen-Funktion. Es ergibt sich eine signifikante per- und postoperative Lymphopenie, wobei sowohl die B- als auch die T-Lymphozyten betroffen sind.

Die oben dargestellte, beispielsweise von Bettina Gräfe in ihrer Dissertation, 2004 aus dem Universitätsklinikum Münster beschriebene, durch große chirurgische Eingriffe verursachte Immunsuppression müsste eigentlich dazu führen, dass die erfindungsgemäß eingesetzten trifunktionellen bispezifischen und trispezifischen Antikörper ihre Wirksamkeit nicht entfalten können. Diese trifunktionellen Antikörper besitzen nämlich nicht nur die Kapazität zur direkten Zerstörung der Tumorzellen, sondern sind auch in der Lage, eine gegen den zu behandelnden Tumor gerichtete Immunität zu induzieren. Sie sind befähigt, durch ihre Bindung an den Fc-Rezeptor eine Fc-Rezeptor positive Zelle zu aktivieren und hierdurch die Expression von Zytokinen und/oder von costimulatorischen Antigenen zu initiieren bzw. zu erhöhen. Diese Zytokine und costimulatorischen Antigene übertragen an die T-Zelle zumindest ein zweites Aktivierungssignal, das für eine physiologische Aktivierung der T-Zelle benötigt wird, und erhöhen damit die Expression von Aktivierungsmarkern, fördern die Proliferation von T-Zellen und zerstören die Tumorzelle.

Die erfindungsgemäß verabreichten trifunktionellen Antikörper besitzen zwei unterschiedliche Bindungsarme und können daher gleichzeitig zwei verschiedene Antigenstrukturen erkennen und binden. Mit einem Bindungsarm bindet der trifunktionelle Antikörper an einem Tumorantigen auf der Tumorzelle, und mit Hilfe der zweiten Antigenstruktur an T-Lymphozyten und führt beide in einen engen räumlichen Kontext, so dass die T-Zelle in die Lage versetzt wird, die Tumorzelle zu zerstören.

Man konnte davon ausgehen, dass diese hochkomplexe Immunantwort, die zu einer Stimulierung des Immunsystems gegen den Tumor und zu einer direkten Zerstörung der Tumorzelle führt, bei der intraoperativen Verabreichung, bevorzugt bei einer direkten Verabreichung intraoperativ direkt lokal in die Bauchhöhle, nicht wirksam ist, und zwar auf Grund der oben beschriebenen immunsuppressiven Wirkung, in welchen der Körper des Patienten durch die Operation versetzt wird, um die "überschießende" Immunantwort durch den abdominalen chirurgischen Eingriff auszugleichen. Der Körper versucht nämlich, spezifische zelluläre Immunfunktionen, beispielsweise die Aktivität von Phagozyten im Bauchraum, zu reprimieren.

Weiterhin versucht der Körper durch Immunsuppression die erhöhte Bildung von Zytokinen und Akute-Phase-Proteinen zu vermindern, um den Körper wieder in eine normale, physiologische Immunsituation zu bringen. Diese postoperative Suppression des Immunsystems ließ erwarten, dass die Verabreichung der trifunktionellen Antikörper (trAk) nicht zu einer Zerstörung intraperitoneal disseminierter Tumorzellen führt. Aus diesem Grund wurden Antikörper bisher auch intraoperativ lokal nicht angewandt, da ein Erfolg nicht absehbar war. Völlig überraschend zeigte sich jedoch, dass die erfindungsgemäß ausgewählten trifunktionellen bispezifischen und trispezifischen Antikörper sehr wohl befähigt sind, trotz der immunsuppressiven Situation ihre immunologischen Funktion wahrzunehmen und eine direkte Zerstörung der Tumorzellen zu bewirken. Diese Funktionen sind insbesondere eine physiologische Aktivierung der T-Zellen, die durch costimulatorische Signale erreicht wird, die von den aktivierten akzessorischen Zellen an die T-Zellen vermittelt werden. Weiterhin werden, neben den T-Zellen, akzessorische Zellen rekrutiert, so dass weitere Killermechanismen wie z. B. eine Phagozytose initiiert werden. Durch diese Mechanismen sind die trifunktionellen Antikörper in der Lage, auch Tumorzellen zu zerstören, welche gegenüber T-Zellen resistent sind. Diese Zerstörung disseminierter Tumorzellen erfolgt trotz der beschriebenen immunsuppressiven Situation nach Tumorresektion in der Bauchhöhle.

Die erfindungsgemäß beanspruchte Verwendung bzw. die erfindungsgemäß beanspruchte, zweckgebundene pharmazeutische Zusammensetzung bzw. das erfindungsgemäß beanspruchte Heilverfahren wird unter Bezugnahme auf den Anspruch 1 nachfolgend näher beschrieben. Bevorzugte Ausgestaltungen der Erfindung ergeben sich aus den Unteransprüchen, der vorliegenden Beschreibung sowie den Ausführungsbeispielen.

Die von der Erfindung umfassten trifunktionellen bispezifischen und trispezifischen Antikörper werden während des chirurgischen Eingriffs im Bauchraum eingesetzt. Zunächst erfolgt eine Eröffnung der Bauchhöhle, d. h. eine Laparotomie mit nachfolgender Tumorresektion durch an sich bekannte operative Techniken. Die trifunktionellen bispezifischen und trispezifischen Antikörper werden also intraoperativ eingesetzt, d. h. während eines chirurgischen Eingriffs bzw. während einer Operation. In einer besonders bevorzugten Ausgestaltungsform der Erfindung erfolgt die Applikation der Antikörper nach der Resektion des Tumormaterials, jedenfalls vor Verschließung der Bauchhöhle durch direkte Applikation in die Bauchhöhle. Die Ausdrücke "Bauchraum" und "Bauchhöhle" werden hier synonym eingesetzt. Der Kern der Erfindung liegt also in der direkten Kontaktierung des trifunktionellen bispezifischen und trispezifischen Antikörpers; wie er in der vorliegenden Anmeldung beschrieben wird, mit der Umgebung der Bauchhöhle und nicht darin, den Antikörper postoperativ zu verabreichen. Lediglich in einer bevorzugten Ausgestaltung der Erfindung wird zusätzlich nach dem chirurgischen Eingriff, d. h. nach Verschluss des Bauchraums, zumindest eine weitere, zweite, dritte usw. Antikörperdosis verabreicht, um eventuell verbliebene, noch nicht zerstörte Tumorzellen zu zerstören.

Bei den Tumoren handelt es sich um Tumoren, die üblicherweise Organe im Bauchraum befallen. Dies sind insbesondere gastrointestinale Tumoren, z. B. Tumoren des Magens, des Dünn- und Dickdarms, der Leber, der Nieren, des Ovars, der Bauchspeicheldrüse, der Appendix und des Pseudoyxoms. Nach Entfernung des Tumors wird der trifunktionelle bispezifische Antikörper oder der trispezifische Antikörper oder eine Mischung beider Antikörper in die Bauchhöhle appliziert. Da der Antikörper möglichst mit allen Oberflächen in der Bauchhöhle so vollständig wie möglich in Kontakt treten soll, wird er in einem physiologisch verträglichen Medium, das zum Spülen der Bauchhöhle geeignet ist, appliziert. Bei einem derartigen Medium handelt es sich bevorzugt um eine isotonische bzw. physiologische Lösung, bevorzugt eine isotonische bzw. physiologische Kochsalzlösung. Selbstverständlich sind alle anderen Medien, die sich zur Aufnahme und zur Verteilung des Antikörpers in der Bauchhöhle eignen und die physiologisch verträglich sind, ebenfalls einsetzbar, beispielsweise Pufferlösungen.

Zum Medium können auch weitere pharmazeutisch aktive Stoffe zugegeben werden, beispielsweise Antibiotika, Antimykotika, Anti-Virus-Mittel, antiinflammatorisch wirkende Mittel, analgetisch oder antipyretisch wirkende Mittel, weitere Anti-Tumor-Mittel wie Zytostatika oder andere Antikörper etc. Auch die zusätzliche Gabe von unspezifischen Immunmodulatoren wie GM-CSF, G-CSF usw. ist möglich. Es bleibt dem Fachmann überlassen, über die weiteren pharmazeutisch aktiven Stoffe zu entscheiden, die der Spülflüssigkeit zugegeben werden. Der Fachmann wird über die Art und die Menge dieser weiteren Wirkstoffe in Verbindung mit der zu behandelnden Erkrankung eine Entscheidung treffen. Zusätzlich können pharmazeutisch inaktive Stoffe beigefügt werden, z. B. Stabilisierungsmittel wie Polysorbate, Konservierungsmittel etc.

Das den Antikörper enthaltende Medium wird bei einer direkten lokalen Applikationsform für einen Zeitraum in der Bauchhöhle verbleiben, der ausreichend ist, um einen vollständigen Kontakt zwischen dem Antikörper und den disseminierten Tumorzellen herzustellen. Dieser Zeitraum wird vom behandelnden Arzt bestimmt werden, der seine Entscheidung in Abhängigkeit von der zu behandelnden Erkrankung, den Zustand des Patienten und weiteren Parametern bestimmen wird. Bevorzugt verbleibt das den Antikörper enthaltende Medium für einen Zeitraum von mehreren Minuten, z. B. 1 - 10 Minuten oder 1 - 5 Minuten oder bis zu einer Stunde, möglicherweise auch länger, in der Bauchhöhle. Besonders bevorzugt verbleibt die Flüssigkeit in der Bauchhöhle, wobei sie dann bevorzugt in einer solchen Menge vorliegt, so dass sie vom Körper resorbierbar ist. Üblicherweise werden Flüssigkeiten vom Körper im Bauchraum nach spätestens 24 Stunden weitgehend resorbiert. Bevorzugt wird also die Flüssigkeit in einer solchen Menge eingesetzt, d. h. intraperitoneal, also in die Bauchhöhle, appliziert, dass sie vom Körper resorbierbar ist.

In einer bevorzugten Ausgestaltung der Erfindung wird die Bauchhöhle zumindest einmal mit dem den Antikörper enthaltenden physiologisch verträglichen Medium in Kontakt gebracht, das heißt der Antikörper wird in einem physiologisch verträglichen Medium in die Bauchhöhle appliziert. Das Einbringen erfolgt zum Beispiel durch eine Spritze in den geöffneten Bauchraum (d. h. nicht durch die geschlossene Bauchdecke), durch Eingießen, oder andere, an sich bekannte Applikationsarten. In einer Ausgestaltungsform der Erfindung wird der Antikörper in Form einer Spülflüssigkeit eingesetzt, um durch die Spülung selbst und dem anschließenden Entfernen der Spülflüssigkeit disseminierte Tumorzellen auszutragen. Diese Spülung kann bevorzugt ein oder zweimal wiederholt werden. Bevorzugt verbleibt aber eine für den Patienten akzeptable Menge an Flüssigkeit nach Applikation in der Bauchhöhle, um einen ausreichend langen Kontakt zwischen dem Antikörper und den disseminierten Tumorzellen zu ermöglichen. Wie oben dargestellt wird das physiologisch verträgliche Medium mit dem Antikörper vom Körper resorbiert werden. Alternativ kann es auch aktiv entfernt werden.

Das physiologisch verträgliche Medium kann auf beliebige, vom Arzt bestimmte Weise in die Bauchhöhle appliziert werden. Beispiele hierfür sind das direkte Eingießen des Mediums, eine Applikation über einen Katheter oder über einen permanent eingesetzten Katheter oder über Schlauchsysteme. Weitere bevorzugte Ausgestaltungen sind perkutan eingesetzte Katheter oder subkutan implantierte Portsysteme.

Die Menge des zu applizierenden Antikörpers wird wiederum vom zu behandelnden Arzt in Abhängigkeit von der zu therapierenden Erkrankung bestimmt. Der Antikörper wird in einer Menge von 1-400 µg pro Applikation verabreicht, bevorzugt in einer Menge von 5-100 µg, weiterhin bevorzugt in einer Menge von 10-20 µg. Weitere einsetzbare Mengen sind 10-50 µg, 20-80 µg bzw. 30-60 µg. Die verabreichten Dosismengen des Antikörpers können auch unterschiedlich gewählt werden. Es ist für einen Fachmann selbstverständlich, dass es sich bei diesen Mengenangaben um Richtwerte handelt, die von Patient zu Patient, von Erkrankung zu Erkrankung und von Antikörper zu Antikörper durchaus unterschiedlich sein können. Es gehört zum Fachwissen eines Mediziners, die individuell einzusetzende Antikörpermenge, beispielsweise in Abhängigkeit davon, wie der Patient auf die Antikörperapplikation reagiert, entsprechend auszuwählen.

Die Applikation der Antikörper erfolgt also erfindungsgemäß während der durch den chirurgischen Eingriff verursachten immunsuppresiven Situation und unterscheidet sich damit signifikant von einer im Stand der Technik üblichen Applikationsart, bei der die Antikörper an den Patienten zwar direkt in die Bachhöhle appliziert werden können, aber keine immunsuppressive Umgebung existiert, da die Anwendung nicht intraoperativerfolgt und der Bachraum auch nicht eröffnet ist.

Der Antikörper wird in einer weiteren Ausgestaltungsform der Erfindung postoperativ bevorzugt in zumindest einer weiteren Dosis verabreicht, beispielsweise in Form einer zweiten Dosis zur Aktivierung und Proliferation der Immunzellen und vorzugsweise zumindest in einer dritten Dosis zur Zerstörung verbliebener residueller und disseminierter Tumorzellen. Die Anzahl der weiteren Applikationen hängt zum Beispiel von der Reaktion des Patienten auf die Antikörperapplikation ab, beispielsweise von den Nebenwirkungen, und wird vom zu behandelnden Arzt bestimmt. Bevorzugte Mengen des zu applizierenden Antikörpers werden ebenfalls vom behandelnden Arzt in Abhängigkeit von der zu behandelnden Erkrankung und vom Antikörper bestimmt. Als Richtwerte kann postoperativ als zweite Dosis eine Menge von 1-20 µg, bevorzugt 5-10 µg, verabreicht werden, eine nachfolgende Dosis bevorzugt in einer Menge von 20-100 µg, bevorzugt 30-60 µg, und weitere nachfolgende Verabreichungen bevorzugt in einer Menge von 100-500 µg, bevorzugt 100-300 µg. Die postoperative Applikation kann sowohl intraperitoneal als auch intravenös oder über eine andere an sich bekannte Applikationsart erfolgen.

In einer weiteren Ausgestaltungsform der Erfindung wird der Antikörper postoperativ in einer Menge von 5-1000 µg, bevorzugt 10-500 µg, weiterhin bevorzugt 10-150 µg verabreicht.

Die Verabreichung erfolgt bevorzugt intraperitoneal, wobei beispielsweise eine postoperative Verabreichung 2 bis 5 mal erfolgt.

Die Verabreichung erfolgt in zeitlichen Abständen, wobei z. B. 48-72 Stunden nach der Operation gewählt werden. Beispielsweise wird zwischen den einzelnen Applikationen ein Zeitraum von 2-3 Tagen plusminus mehrere Stunden gewählt. Die Applikationen werden bevorzugt postoperativ über einen Zeitraum von 12-20 Tagen fortgesetzt. Bei Verwendung der erfindungsgemäßen, z. B. murinen Antikörper wird beispielsweise die HAMA (human anti mouse antibody)-Reaktion bestimmt, die im Allgemeinen nach 16-21 Tagen einsetzt, bei einigen Patienten früher, bei anderen später oder überhaupt nicht. Aber selbst eine beginnende HAMA-Reaktion muss die Wirksamkeit während dieses Zeitraums nicht notwendigerweise beeinflussen, da hier u.U. noch keine ausreichenden HAMA-Spiegel erreicht werden. Die bisherigen Erfahrungen zeigen, dass durchaus auch hohe HAMA-Spiegel toleriert werden können, ohne dass relevante Nebenwirkungen oder eine deutliche Abschwächung der Effektivität der erfindungsgemäß eingesetzten trifunktionellen Antikörper beobachtet wurden.

Die erfindungsgemäß eingesetzten trifunktionellen bispezifischen und/oder trispezifischen Antikörper sind an sich bekannt. Es handelt sich um Antikörper mit den nachfolgenden Eigenschaften:
(a) bindet an eine T-Zelle;
(b) bindet an zumindest ein tumorassoziiertes Antigen, welches auf intraperitoneal vorkommenden Tumorzellen exprimiert wird;
(c) bindet durch seinen Fc-Teil (bei trifunktionellen bispezifischen Antikörpern) oder durch ein dritte Spezifität (bei trispezifischen Antikörpern) an Fc-Rezeptor positive Zellen.

Bei den Antikörpern handelt es sich um sogenannte intakte trifunktionelle bispezifische und trispezifische Antikörper. Durch die Behandlung wird nicht nur die oben beschriebene überraschende direkte Zerstörung der Tumorzellen bei einer intraoperativen Applikation während der immunsuppressiven Phase erreicht, sondern insbesondere durch die nachfolgenden Gaben kann auch eine gegen den zu behandelnden Tumor gerichtete Immunität induziert werden.

Unter "intakte Antikörper" sind solche zu verstehen, die einen funktionellen Fc-Teil besitzen. Bevorzugt handelt es sich hierbei um heterologe Antikörper, d.h. sie sind aus schweren Immunglobulinketten unterschiedlicher Subklassen (-kombinationen, auch Fragmente) und/oder Herkunft (Spezies) zusammengesetzt.

Neben den oben beschriebenen Merkmalen a, b und c weisen die eingesetzten Antikörper in bevorzugten Ausgestaltungen der Erfindung noch die weiteren Merkmale d) und e) auf:
d) aktiviert die Fc-Rezeptor positive Zelle durch seine Bindung an die Fc-Rezeptor positive Zelle, wodurch die Expression von Cytokinen und/oder von kostimulatorischen Antigenen initiiert oder erhöht wird;
e) die kostimulatorischen Antigene und/oder Cytokine übertragen an die T-Zelle zumindest ein 2. Aktivierungssignal, das für eine physiologische Aktivierung der T-Zelle benötigt wird, wobei sich diese Aktivierung in der Hochregulation von Aktivierungsmarkern, der Zerstörung der Tumorzelle und/oder in einer Proliferation der T-Zelle zeigt.

Die Erfindung wird nachfolgend insbesondere am Beispiel trifunktioneller bispezifischer Antikörper beschrieben. Die Resultate sind aber auch mit trispezifischen Antikörpern erzielbar.

Die erfindungsgemäß verwendbaren Antikörper sind befähigt, die Fc-Rezeptor positive Zelle zu aktivieren, wodurch die Expression von Zytokinen und/oder kostimulatorischen Antigenen initiiert oder erhöht wird.

Bei den trispezifischen Antikörpern erfolgt die Bindung an die Fc-Rezeptor positiven Zellen bevorzugt beispielsweise über den Fc-Rezeptor von Fc-Rezeptor positiven Zellen oder auch über andere Antigene auf Fc-Rezeptor positiven Zellen (Antigen-präsentierenden Zellen), wie z.B. den Mannose-Rezeptor.

Die erfindungsgemäß verwendbaren heterologen bispezifischen und/oder trispezifischen Antikörper sind an sich bekannt. Sie sind zum Beispiel beschrieben in Lindhofer et al., Blood, 88:4651, 1996 oder Lindhofer et al., J. Immunology, 155:219, 1995.

Auf der Tumorzelle erfolgt eine Hochregulation von MHC 1, sowie eine Aktivierung der intrazellulären Prozessierungsmaschinerie (Proteasom-Komplex) aufgrund der Freisetzung von Zytokinen (wie z.B. INF-γ und TNF-α) in unmittelbarer Nachbarschaft der Tumorzelle. Die Zytokine werden aufgrund trifunktioneller Antikörper-vermittelter Aktivierung von T-Zellen und akzessorischen Zellen freigesetzt. D.h. durch den intakten bsAk werden nicht nur Tumorzellen zerstört oder phagozytiert, sondern indirekt auch deren Tumorimmunogenität erhöht.

Die Aktivierung der Fc-Rezeptor positiven Zelle durch den trAk ist von der Subklasse bzw. der Subklassenkombination des trAk abhängig. Wie in in vitro-Versuchen nachgewiesen werden konnte, sind beispielsweise trAk der Subklassenkombination Maus-IgG2a/Ratte-IgG2b in der Lage, an Fc-Rezeptor positive Zellen zu binden und diese gleichzeitig zu aktivieren, was zur Hochregulation bzw. Neuausbildung (Expression) von kostimulatorischen Antigenen, wie z.B. CD40, CD80 oder CD86, auf der Zelloberfläche dieser Zellen führt (Zeidler et al., J. Immunol., 163:1246, 1999). Dagegen sind bsAk der Subklassenkombination Maus-IgG1/RatteIgG2b zwar in der Lage an Fc-Rezeptor positive Zellen zu binden (Haagen et al., J. Immunology, 1995, 154: 1852-1860), können diese aber offenbar nicht in vergleichbarem Maße aktivieren (Gast et al., Cancer Immunol. Immuntherap., 1995, 40: 390).

Während der intakte trAk die T-Zelle mit einem Bindungsarm (z.B. an CD3 oder CD2) bindet und gleichzeitig aktiviert, können kostimulatorische Signale von der an den Fc-Teil des trAk gebundenen Fc-Rezeptor positiven Zelle an die T-Zelle übermittelt werden. D.h. erst die Kombination von Aktivierung der T-Zelle über einen Bindungsarm des trAk und der gleichzeitigen Übertragung von kostimulatorischen Signalen von der Fc-Rezeptor positiven Zelle auf die T-Zelle, führt zu einer effizienten T-Zellaktivierung. Tumor-spezifische T-Zellen, die an der Tumorzelle ungenügend aktiviert wurden und anergisch sind, können nach der erfindungsgemäßen Behandlung mit intakten bispezifischen Antikörpern oder trispezifischen Antikörpern ebenfalls reaktiviert werden.

Ein weiterer wichtiger Aspekt ist die mögliche Phagozytose, Prozessierung und Präsentation von Tumorbestandteilen durch die vom trAk herangeführten und aktivierten akzessorischen Zellen (Monozyten/Makrophagen, oder dendritische Zellen). Durch diesen klassischen Mechanismus der Präsentation von Antigenen können sowohl tumorspezifische CD4- wie auch CD8-positive Zellen generiert werden. Tumorspezifische CD4-Zellen spielen darüber hinaus eine wichtige Rolle für die Induktion einer humoralen Immunantwort im Zusammenhang mit der T-B-Zell-Kooperation.

Trifunktionale und trispezifische Antikörper können mit einem Bindungsarm an den T-Zellrezeptor-Komplex der T-Zelle, mit dem zweiten Bindungsarm an tumorassoziierte Antigene auf der Tumorzelle binden. Sie aktivieren dabei T-Zellen, die durch Freisetzung von Zytokinen oder Apoptose-vermittelnde Mechanismen die Tumorzellen zerstören. Darüber hinaus besteht offenbar die Möglichkeit, daß T-Zellen im Rahmen der Aktivierung mit bispezifischen Antikörpern tumorspezifische Antigene über ihren Rezeptor erkennen und dadurch eine dauerhafte Immunisierung eingeleitet wird. Von besonderer Bedeutung ist dabei der intakte Fc-Teil des trifunktionalen oder trispezifischen Antikörpers, der die Bindung an akzessorische Zellen wie z.B. Monozyten/Makrophagen und dendritische Zellen vermittelt und diese veranlaßt selbst zytotoxisch zu werden und/oder gleichzeitig wichtige kostimulatorische Signale an die T-Zelle weiterzugeben. Auf diese Weise kann offensichtlich eine T-Zellantwort u.U. auch gegen bislang unbekannte, tumorspezifische Peptide induziert werden.

Durch Redirektion von u. U. anergisierten, tumorspezifischen T-Zellen an Tumorzellen mittels trifunktionaler und/oder trispezifischer Antikörper bei gleichzeitiger Kostimulation derartiger T-Zellen durch akzessorische Zellen welche an den Fc-Teil des trifunktionalen oder trispezifische Antikörpers binden, könnte die Anergie von zytotoxischen T-Zellen (CTLs) aufgehoben werden. D.h. eine im Patienten gegen den Tumor existierende T-Zell-Toleranz kann mittels intakter heterologer trifunktionaler und/oder trispezifischer Antikörper gebrochen und damit eine dauerhafte Tumorimmunität induziert werden.

Die erfindungsgemäß eingesetzten Antikörper sind bevorzugt zur Reaktivierung von in Anergie befindlichen, tumorspezifischen T-Zellen befähigt. Weiterhin sind sie zur Induktion von tumorreaktiven komplement-bindenden Antikörpern und damit zur Induktion einer humoralen Immunantwort in der Lage.

Die Bindung erfolgt bevorzugt über CD3, CD2, CD4, CD5, CD6, CD8, CD28, CD40L und/oder CD44 an die T-Zelle. Die Fc-Rezeptor positiven Zellen weisen zumindest einen Fcγ-Rezeptor Typ I oder III auf.
Erfindungsgemäß einsetzbare Antikörper sind zur Bindung an Monozyten, Makrophagen, dendritische Zellen, "Natural Killer"-Zellen (NK-Zellen) und/oder aktivierte neutrophile Zellen als Fcγ-Rezeptor I und/oder III positive Zellen befähigt (Zeidler, 1999, Zeidler 2000 a.a.o.).

Die erfindungsgemäß einsetzbaren Antikörper bewirken, daß die Expression von CD40, CD80, CD86, ICAM-1 und/oder LFA-3 als kostimulatorische Antigene oder/und die Sekretion von Zytokinen durch die Fc-Rezeptor positive Zelle initiiert oder erhöht wird. Die Zytokine sind bevorzugt IL-1, IL-2, IL-4, IL-6, IL-8,IL-12, INF-γ und/oder TNF-α.

Die erfindungsgemäß einsetzbaren trifunktionalen Antikörper sind beispielsweise:
- ein anti-CD3 X anti-Tumor-assoziiertes Antigen- und/oder anti-CD4 X anti-Tumor-assoziiertes Antigen- und/oder anti-CD5 X anti-Tumor-assoziiertes Antigen- und/oder anti-CD6 X anti-Tumor-assoziiertes Antigen- und/oder anti-CD8 X anti-Tumor-assoziiertes Antigen- und/oder anti-CD2 X anti-Tumor-assoziiertes Antigen- und/oder anti-CD28 X anti-Tumor-assoziiertes Antigen- und/oder anti-CD40L X anti-Tumor-assoziiertes Antigen-Antikörper und/oder anti-CD44 X anti-Tumor-assoziiertes Antigen-Antikörper.

Die erfindungsgemäß einsetzbaren trispezifischen Antikörper sind bevorzugt:
- ein anti-CD3 X anti-Tumor-assoziiertes Antigen- und/oder anti-CD4 X anti-Tumor-assoziiertes Antigen- und/oder anti-CD5 X anti-Tumor-assoziiertes Antigen- und/oder anti-CD6 X anti-Tumor-assoziiertes Antigen- und/oder anti-CD8 X anti-Tumor-assoziiertes Antigen- und/oder anti-CD2 X anti-Tumor-assoziiertes Antigen- und/oder anti-CD28 X anti-Tumor-assoziiertes Antigen- und/oder anti-CD40L X anti-Tumor-assoziiertes Antigen-Antikörper und/oder anti-CD44 X anti-Tumor-assoziiertes Antigen-Antikörper.

Die erfindungsgemäß einsetzbaren Antikörper erkennen und binden an sich bekannte tumor-assoziierte Antigene, die typischerweise auf Tumorzellen im Bauchraum vorkommen. Bevorzugt werden von den Antikörpern tumor-assoziierte Antigene auf einer Tumorzelle erkannt, die der nachfolgenden Gruppe angehören: Her2/neu, CD20, CD30 EpCAM, G250, Proteoglycane, GD2, GD3, MHC II, EGF-R, CA125 und CEA.

Bei dem Antikörper handelt es sich bevorzugt um nachfolgende Antikörper: ein anti-Her2/neu x anti-CD3 Antikörper oder ein anti-EpCAM x anti-CD3-Antikörper ist, der an Fc-γ-Typ I/III-Rezeptoren bindet, jeweils bevorzugt mit der Isotypkombination Ratte-IgG2b/Maus-IgG2a oder Maus-[VH-CH1, VL-CL]-human IgG1-[hinge]- human-IgG3 [Caucasian allotypes G3m(b+g)= no binding to protein A)-[CH2-CH3] /Ratte-[VH-CH1, VL-CL]-human IgG1-[hinge]- human IgG1-[CH2-CH3].

Die erfindungsgemäß einsetzbaren trispezifischen Antikörper weisen zumindest eine T-Zell-Bindungsarm, einen Tumorzell-Bindungsarm und einen an Fc-Rezeptor positive Zellen bindenden Bindungsarm auf. Dieser zuletzt genannte Bindungsarm kann ein anti-Fc-Rezeptor-Bindungsarm oder ein Mannose-Rezeptor-Bindungsarm sein.

Der trifunktionale Antikörper ist bevorzugt ein heterologer intakter Ratte/Maus bispezifischer Antikörper.

Mit den erfindungsgemäß einsetzbaren trifunktionalen und trispezifischen Antikörpern werden T-Zellen aktiviert und gegen die Tumorzellen redirigiert. Bevorzugt einsetzbare heterologe intakte bispezifische Antikörper werden aus einer oder mehreren der nachfolgenden Isotyp-Kombinationen ausgewählt:
Ratte-IgG2b/Maus-IgG2a,
Ratte-IgG2b/Maus-IgG2b,
Ratte-IgG2b/Maus-IgG3,

Ratte-IgG2b/Human-IgG1,
Ratte-IgG2b/Human-IgG2,
Ratte-IgG2b/Human-IgG3[orientaler Allotyp G3m(st) = Bindung an Protein A],
Ratte-IgG2b/Human-IgG4,

Ratte-IgG2b/Ratte-IgG2c,
Maus-IgG2a/Human-IgG3[kaukasische Allotypen G3m(b+g) = keine Bindung an Protein A, im folgenden mit * gekennzeichnet]

Maus-IgG2a/Maus-[VH-CH1,VL-CL]-Human-IgG1-[Hinge]-Human-IgG3*-[CH2-CH3]
Maus-IgG2a/Ratte-[VH-CH1,VL-CL]-Human-IgG1-[Hinge]-Human-IgG3*-[CH2-CH3]
Maus-IgG2a/Human-[VH-CH1,VL-CL]-Human-IgG1-[Hinge]-Human-IgG3*-[CH2-CH3]
Maus-[VH-CH1,VL-CL]-Human-IgG1/Ratte-[VH-CH1,VL-CL]-Human-IgG1-[Hinge]-Human-IgG3*-[CH2-CH3]
Maus-[VH-CH1,VL-CL]-Human-IgG4/Ratte-[VH-CH1,VL-CL]-Human-IgG4-[Hinge]-Human-IgG4[N-terminale Region von CH2]-Human-IgG3*[C-terminale Region von CH2: > AS-Position 251]-Human-IgG3*[CH3]
Ratte-IgG2b/Maus-[VH-CH1,VL-CL]-Human-IgG1-[Hinge-CH2-CH3]
Ratte-IgG2b/Maus-[VH-CH1,VL-CL]-Human-IgG2-[Hinge-CH2-CH3]
Ratte-IgG2b/Maus-[VH-CH1,VL-CL]-Human-IgG3-[Hinge-CH2-CH3, orientaler Allotyp]
Ratte-IgG2b/Maus-[VH-CH1,VL-CL]-Human-IgG4-[Hinge-CH2-CH3]
Human-IgG1/Human-[VH-CH1,VL-CL]-Human-IgG1-[Hinge]-Human-IgG3*-[CH2-CH3]
Human-IgG1/Ratte-[VH-CH1,VL-CL]-Human-IgG1-[Hinge]-Human-IgG4[N-terminale Region von CH2]-Human-IgG3*[C-terminale Region von CH2 : > AS-Position 251]-Human-IgG3*[CH3]
Human-IgG1/Maus-[VH-CH1,VL-CL]-Human-IgG1-[Hinge]-Human-IgG4[N-terminale Region von CH2]-Human-IgG3*[C-terminale Region von CH2 : > AS-Position 251]-Human-IgG3*[CH3]
Human-IgG1/Ratte-[VH-CH1,VL-CL]-Human-IgG1-[Hinge]-Human-IgG2[N-terminale Region von CH2]-Human-IgG3*[C-terminale Region von CH2 : > AS-Position 251]-Human-IgG3*[CH3]
Human-IgG1/Maus-[VH-CH1,VL-CL]-Human-IgG1-[Hinge]-Human-IgG2[N-terminale Region von CH2]-Human-IgG3*[C-terminale Region von CH2 : > AS-Position 251]-Human-IgG3*[CH3]
Human-IgG1/Ratte-[VH-CH1,VL-CL]-Human-IgG1-[Hinge]-Human-IgG3*-[CH2-CH3]
Human-IgG1/Maus-[VH-CH1,VL-CL]-Human-IgG1-[Hinge]-Human-IgG3*-[CH2-CH3]
Human-IgG2/Human-[VH-CH1,VL-CL]-Human-IgG2-[Hinge]-Human-IgG3*-[CH2-CH3]
Human-IgG4/Human-[VH-CH1,VL-CL]-Human-IgG4-[Hinge]-Human-IgG3*-[CH2-CH3]
Human-IgG4/Human-[VH-CH1,VL-CL]-Human-IgG4-[Hinge]-Human-IgG4[N-terminale Region von CH2]-Human-IgG3*[C-terminale Region von CH2 : > AS-Position 251]-Human-IgG3*[CH3]
Maus-IgG2b/Ratte-[VH-CH1,VL-CL]-Human-IgG1-[Hinge]-Human-IgG3*-[CH2-CH3]
Maus-IgG2b/Human-[VH-CH1,VL-CL]-Human-IgG1-[Hinge]-Human-IgG3*-[CH2-CH3]
Maus-IgG2b/Maus-[VH-CH1,VL-CL]-Human-IgG1-[Hinge]-Human-IgG3*-[CH2-CH3]
Maus-[VH-CH1,VL-CL]-Human-IgG4/Ratte-[VH-CH1,VL-CL]-Human-IgG4-[Hinge]-Human-IgG4-[CH2]-Human-IgG3*-[CH3]
HumanIgG1/Ratte[VH-CH1,VL-CL]-Human-IgG1[Hinge]-Human-IgG4-[CH2]-HumanIgG3*[CH3]
HumanIgG1/Maus[VH-CH1,VL-CL]-Human-IgG1[Hinge]-Human-IgG4-[CH2]-Human-IgG3*[CH3]
Human-IgG4/Human[VH-CH1,VL-CL]-Human-IgG4-[Hinge]-Human-IgG4-[CH2]-HumanIgG3*-[CH3]

Bei den erfindungsgemäß verwendbaren Antikörpern handelt es sich vorzugsweise um monoklonale, chimäre, rekombinante, synthetische, halbsynthetische oder chemisch modifizierte intakte oder nicht-intakte Antikörper mit beispielsweise Fv-, Fab-, scFv- oder F(ab)₂-Fragmenten.

Bevorzugt werden Antikörper oder Derivate oder Fragmente vom Menschen verwendet oder solche, die derart verändert sind, daß sie sich für die Anwendung beim Menschen eignen (sogenannte "humanized antibodies") (siehe z.B. Shalaby et al., J. Exp. Med. 175 (1992), 217; Mocikat et al., Transplantation 57 (1994), 405).

Die Herstellung der verschiedenen oben genannten Antikörpertypen und -fragmente ist dem Fachmann geläufig. Die Herstellung monoklonaler Antikörper, die ihren Ursprung vorzugsweise in Säugetieren, z.B. Mensch, Ratte, Maus, Kaninchen, Ziege oder Kamelartigen, haben, kann mittels herkömmlicher Methoden erfolgen, wie sie z.B. in Köhler und Milstein (Nature 256 (1975), 495), in Harlow und Lane (Antibodies, A Laboratory Manual (1988), Cold Spring Harbor) oder in Galfré (Meth. Enzymol. 73 (1981), 3) oder der DE 195 31 346 beschrieben sind.

Ferner ist es möglich, die beschriebenen Antikörper mittels rekombinanter DNA-Technologie nach dem Fachmann geläufigen Techniken herzustellen (siehe Kurucz et al., J. Immunol. 154 (1995), 4576; Holliger et al., Proc. Natl. Acad. Sc. USA 90 (1993), 6444).

Die Herstellung von Antikörpern mit zwei verschiedenen Spezifitäten, den sogenannten bispezifischen Antikörpern, ist zum einen durch Anwendung rekombinanter DNA-Technologie möglich, aber auch durch die sogenannte Hybrid-Hybridoma-Fusionstechnik (siehe z.B. Milstein et al., Nature 305 (1983), 537). Hierbei werden Hybridomazellinien, die Antikörper mit jeweils einer der gewünschten Spezifitäten produzieren, fusioniert und Zellklone (Quadrome) identifiziert und isoliert, die Antikörper mit beiden Spezifitäten produzieren.

Das der Erfindung zugrunde liegende Problem kann sowohl durch trifunktionale bispezifische als auch trispezifische Antikörper gelöst werden, die bevorzugt die gekennzeichneten Eigenschaften und Wirkungen aufweisen. Nachfolgend wird die Herstellung von Antikörpern mit zwei und drei Spezifitäten näher beschrieben. Die Bereitstellung derartiger trifunktionaler und trispezifischer Antikörper gehört zum Stand der Technik, und auf die derartige Herstellungstechniken beschreibende Literatur wird hier voll inhaltlich Bezug genommen.

Die Herstellung von Antikörpern mit drei Spezifitäten, sogenannten trispezifischen Antikörpern, durch die das der Erfindung zugrundeliegende Problem ebenfalls lösbar ist, kann beispielsweise derart erfolgen, daß an eine der schweren Ig-Ketten eines bispezifischen Antikörpers eine dritte Antigenbindungsstelle mit einer weiteren Spezifität, z.B. in Form eines "single chain variable fragments" (scFv), angekoppelt wird. Das scFv kann beispielsweise über einen

-S-S(G₄S)ₙD-I-Linker

an eine der schweren Ketten gebunden sein (S = Serin, G = Glycin, D = Aspartat, I = Isoleucin).

Analog dazu können trispezifische F(ab)₂-Konstrukte hergestellt werden, indem die CH2-CH3-Regionen der schweren Kette einer Spezifität eines bispezifischen Antikörpers ersetzt werden durch ein scFv mit einer dritten Spezifität, während die CH2-CH3-Regionen der schweren Kette der anderen Spezifität beispielsweise durch Einbau eines Stopcodons (am Ende der "Hinge"-Region) in das codierende Gen, z.B. mittels homologer Rekombination, entfernt werden.

Möglich ist auch die Herstellung trispezifischer scFv-Konstrukte. Hierbei werden drei VH-VL-Regionen, die drei verschiedene Spezifitäten repräsentieren, hintereinander in Reihe angeordnet.

Erfindungsgemäß werden z.B. intakte bispezifische Antikörper verwendet. Intakte bispezifische Antikörper sind aus zwei Antikörper-Halbmolekülen (je eine H- und L-Immunglobulinkette), die jeweils eine Spezifität repräsentieren, zusammengesetzt, und besitzen darüber hinaus, wie normale Antikörper auch, einen Fc-Teil mit den bekannten Effektorfunktionen. Sie werden bevorzugt durch die Quadrom-Technologie hergestellt. Dieses Herstellungsverfahren ist beispielhaft in der DE-A-44 19 399 beschrieben. Auf diese Druckschrift, auch bzgl. einer Definition der bispezifischen Antikörper, wird zur vollständigen Offenbarung vollinhaltlich Bezug genommen. Selbstverständlich sind auch andere Herstellungsverfahren einsetzbar, solange sie zu den erfindungsgemäß notwendigen intakten bispezifischen Antikörpern der obigen Definition führen.

Beispielsweise können gemäß dem Herstellungsverfahren von Lindhofer et al., J. Immunology 1995, 155: 219, intakte bispezifische Antikörper in ausreichender Menge produziert werden. Die Kombination von 2 bispezifischen Antikörpern gegen 2 unterschiedliche tumorassoziierte Antigene (z.B. c-erb-B2, und EpCAM) auf den Mammakarzinomzellen minimiert die Gefahr, daß Tumorzellen, die nur ein Antigen exprimieren, unerkannt bleiben.

Weitere Vorteile von intakten bsAk mit der Fähigkeit zur Redirektion von T-Zellen gegenüber den o.g. bsF(ab')2 Fragmenten sind im einzelnen:
1. An intakte trAk können Fc-Rezeptor positive Zellen binden und einerseits über ADCC (antibody-dependent cell-mediated cytotoxicity) direkt zur Tumorzerstörung beitragen sowie andererseits wie oben näher ausgeführt zur T-Zellaktivierung.
2. Durch intakte T-Zell-redirigierende trAk werden auch anergisierte tumorspezifische T-Zellen an die Tumorzelle herangeführt, die erfindungsgemäß direkt am Tumor reaktiviert werden können. Dies kann durch ein bsF(ab')2-Fragment mit den Spezifitäten anti-CD64Xanti-tumorassoziiertes Antigen nicht erreicht werden.

Die Bindung des trAk an Fcγ-RI besitzt zwei wesentliche Vorteile im Hinblick auf eine optimale anti-Tumorwirksamkeit:
(1) Fcγ-RI positive Zellen besitzen die Fähigkeit mittels ADCC Tumorzellen zu eliminieren und können insofern synergistisch zur anti-Tumorwirkung der durch den trAk an die Tumorzelle herangeführten cytotoxischen T-Zellen beitragen.
(2) Fcγ-RI positive Zellen (wie z.B. Monozyten/Makrophagen/Dendriten) sind in der Lage, wichtige kostimulatorische Signale, ähnlich wie bei der Antigen-Präsentation, der T-Zelle zu liefern und damit eine Anergisierung der T-Zelle zu verhindern. Es können weiterhin, als erwünschtes Nebenprodukt, aufgrund der durch intakten trAk vermittelten Interaktion von T-Zelle mit akzessorischer Zelle und Tumorzelle sogar T-Zellen, deren T-Zellrezeptor tumorspezifische Peptide (über MHC Antigene auf der Tumorzelle präsentiert) erkennt, stimuliert werden. Die für eine korrekte Aktivierung der T-Zelle notwendigen Kostimuli würden in dieser Konstellation von der akzessorischen Zelle (z.B. Monocyt) geliefert werden. Insofern sollte der erfindungsgemäße Antikörper neben der direkten T-Zellrezeptorunabhängigen, durch trAk vermittelten Tumorzerstörung ebenfalls tumorspezifische T-Zellen aktivieren und generieren, die nach Abbau der trAk weiterhin im Patienten patrouillieren. D.h. mittels intakter trAk kann ähnlich wie bei gentherapeutischen Ansätzen (z.B. durch Einbau von kostimulatorischen Antigenen wie B-7 in die Tumorzelle) die Tumortoleranz im Patienten durchbrochen werden.

Nachfolgend wird die Erfindung anhand von Ausführungsbeispielen näher erläutert, die erfreulicherweise die überraschend positiven Eigenschaften des Einsatzes trifunktioneller bispezifischer Antikörper zeigen. Die Verwendbarkeit der oben beschriebenen trispezifischen Antikörper wird durch diese Versuche gestützt, die im vollen Umfang hierauf übertragbar sind.

### 1. Ausführungsbeispiel

Ein 59-jähriger Patient war bei einem ausgeprägten Magenkarzinom operiert worden. Bereits 2 Monate zuvor war das Magenkarzinom mit Peritonealkarzinose diagnostiziert worden, und es war primär eine Chemotherapie (PLF-Schema) durchgeführt worden. Jetzt hatte der Patient zunehmend über eine Magenausgangsstenose geklagt, so dass aufgrund dessen die palliative Magenresektion indiziert worden war. Gleichzeitig war besprochen worden, im Sinne eines Heilversuchs bereits intraoperativ mit der Antikörperapplikation zu beginnen. Nach kompletter Entfernung des Magens mit Kolon transversum und Milz erfolgte dann die intraoperative Installation des Antikörpers. Zur Applikation wurden 100 µg des trifunktionalen Antikörpers Ertumaxomab mit den Merkmalen anti-Her2/neu X anti-CD3 (Isotypkombination MausIgG2a x RatteIgG2b) in 500 ml NaCl-Lösung aufgenommen und intraperitoneal appliziert. Zusätzlich war noch ein intraabdominelles Portsystem implantiert worden, über das dann ab dem 11. postoperativen Tag insgesamt 4 x Antikörper appliziert worden war. Als Indiz für eine Wirksamkeit der Antikörper wurde nach intraoperativer Antikörperapplikation die Wundflüssigkeit/Spülflüssigkeit gesammelt und erneut auf Tumorzellen immunzytochemisch überprüft. Es zeigte sich hier kein Anhalt mehr für Tumorzellen, so dass dies als Wirksamkeitsnachweis dieses Konzeptes betrachtet werden kann.

Der Patient überlebte die Operation trotz dieses extrem fortgeschrittenen Tumorstadiums über 9 Monate, was bei einer mittleren Überlebenszeit von erwarteten 3 Monaten ein sehr positives Ergebnis war. Hinzu kam, dass der Patient über 6 Monate ohne Zeichen für eine wesentliche Passagestörung im Gastrointestinaltrakt lebte.

### 2. Ausführungsbeispiel

Im zweiten Heilversuch wurde eine 35-jährige Patientin mit einem sehr ausgedehnten Magenkarzinom operiert und intraoperativ erneut eine Applikation trifunktionaler Antikörper direkt nach Resektion des Tumors vorgenommen. Bei der Patientin war 4 Tage vor der Operation zur histologischen Sicherung eine diagnostische Laparoskopie durchgeführt worden.

Die intraoperative Lavage direkt nach Laparotomie zeigte allerdings reichlich Tumorzellen, so dass beschlossen wurde, intraoperativ die Antikörperapplikation zu versuchen. Zuvor war nachgewiesen worden, dass die Tumorzellen als Zielantigen Her2/neu exprimieren. Diese ausgiebige Operation umfasste eine Gastrektomie, eine Hemikolektomie, eine Splenektomie und Cholecystektomie. Die intraoperative Antikörperapplikation (10µg Ertumaxomab) wurde von der Patientin problemlos vertragen, so dass sie sich postoperativ erwartungsgemäß erholte. Es wurde beschlossen, am 8. postoperativen Tag die Antikörpertherapie intravenös fortzusetzen. Hierzu wurden 6 µg Ertumaxomab i.v. appliziert, welches allerdings nach 2 Stunden bei einem plötzlichem, raschen Fieberanstieg abgebrochen werden musste.

Trotz Absetzens der Therapie zeigte die Patientin am Folgetag einen erneuten Fieberanstieg, so dass in der weiteren Diagnostik sich als Ursache eine Pneumonie im linken Unterlappen darstellte. Diese wurde dann antibiotisch behandelt. Die Patientin wurde dann nach weiteren 10 Tagen erneut mit 10 µg des Antikörpers Ertumaxomab i.p. behandelt. Der Verlauf war problemlos und wurde von der Patientin gut verkraftet. 2 Tage später wurde eine weitere i.p.-Therapie mit dem gleichen Antikörper auf 100 µg gesteigert. Auch diese Applikation wurde problemlos vertragen, so dass die Patientin am Folgetag entlassen werden konnte.

Die zytologische Untersuchung der Wundflüssigkeit am 3. postoperativen Tag hatte eine vollständige Abreicherung von Tumorzellen gezeigt, welches als Anhalt für die Wirksamkeit der intraoperativen Antikörperapplikation bewertet

### 3. Ausführungsbeispiel

### Versuchsansatz:

Patientin mit Magen-Ca
Enzymatische Isolation von autologen Tumorzellen aus dem Primärtumor Isolation von PBMC direkt nach Tumorresektion während der OP

### Stimulationsansatz:

50 µg trifunktionale Antikörper / ml Medium
anti-EpCAM X anti-CD3 (Isotypkombination MausIgG2a x RatteIgG2b)
1,0 x 10e6 PBMC / ml
5,0 x 10e4 autologe Tumorzellen in Suspension während Stimulation Stimulation über 12 h

### Messmethode:

Direkter Zytotoxizitäts-Assay (BCECF-Fluoreszenz-Release-Assay) Inkubationszeit 12h

### Interpretation:

PBMCs wurden während der OP nach Resektion des Magen-Ca entnommen (=Zeitpunkt der operationsbedingten Immunsuppression). Gleichzeitig Herstellung einer autologen Einzelzell-Suspension. Nach Stimulation mit trAk und Targetzellen sind die während der OP entnommenen PBMCs in der Lage, autologe Tumorzellen effektiv zu zerstören. Allogene EpCAM-negative Tumorzellen werden hingegen nicht zerstört. Dieser Effekt funktioniert überraschenderweise direkt nach der Opertion, also in einer immunsuppressiven Situation

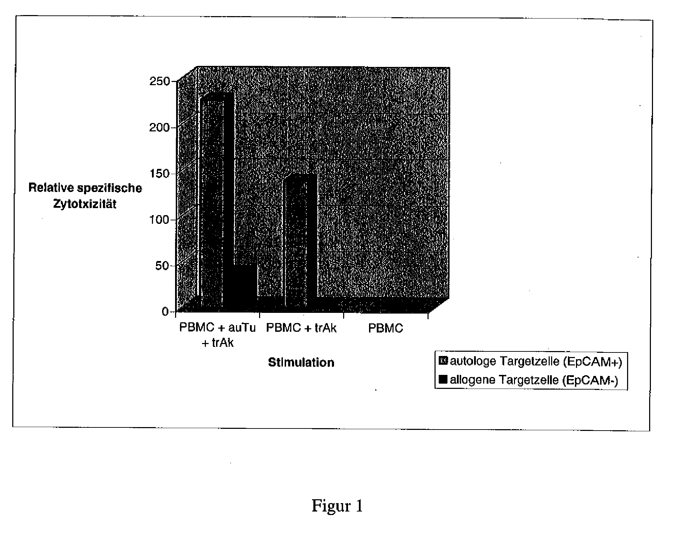

Die beiliegende Figur 1 veranschaulicht diese Wirkungen. Die Figur zeigt:

### Stimulationsansätze:

PBMC + tr. Antikörper + autologe EpCAM+ Targetzellen
PBMC + tr. Antikörper
PBMC

## Patentansprüche

1. Verwendung eines trifunktionellen bispezifischen und/oder trispezifischen Antikörpers mit den nachfolgenden Eigenschaften:
(a) bindet an eine T-Zelle;
(b) bindet an zumindest ein tumorassoziiertes Antigen, welches auf intraperitoneal vorkommenden Tumorzellen exprimiert wird;
(c) bindet durch seinen Fc-Teil (bei bispezifischen Antikörpern) oder durch ein dritte Spezifität (bei trispezifischen Antikörpern) an Fc-Rezeptor positive Zellen
zur Herstellung einer pharmazeutischen Zusammensetzung zur Zerstörung von intraperitoneal disseminierten Tumorzellen und zur Prophylaxe und Therapie von durch intraperitoneal disseminierten Tumorzellen verursachten Tumorrezidiven, wobei der Antikörper intraoperativ, bevorzugt direkt lokal in die Bauchhöhle, verabreicht wird.

2. Verwendung nach Anspruch 1, **dadurch gekennzeichnet, dass** der Antikörper in einem physiologisch verträglichen Medium enthalten ist, das zur Applikation in die Bauchhöhle ausgelegt ist.

3. Verwendung nach Anspruch 1 oder 2, **dadurch gekennzeichnet, dass** das den Antikörper enthaltende Medium für einen Zeitraum in der Bauchhöhle verbleibt, der ausreichend ist, um einen Kontakt zwischen dem Antikörper und disseminierten Tumorzellen herzustellen und das Medium mit dem Antikörper anschließend wieder entfernt wird, oder das Medium mit dem Antikörper in der Bauchhöhle verbleibt und dort resorbiert wird.

4. Verwendung nach einem oder mehreren der vorhergehenden Ansprüche, **dadurch gekennzeichnet, dass** der Antikörper in einer Menge von 1 - 400 µg verabreicht wird, bevorzugt 5 - 100 µg, weiterhin bevorzugt 10 - 50 µg.

5. Verwendung nach einem oder mehreren der vorhergehenden Ansprüche, **dadurch gekennzeichnet, dass** der Antikörper gegen ein Tumorantigen gerichtet ist, ausgewählt aus der Gruppe, bestehend aus Her2/neu, CD20, CD30 EpCAM, G250, Proteoglycane, GD2, GD3, MHC II, EGF-R, CA125 und CEA.

6. Verwendung nach einem oder mehreren der vorhergehenden Ansprüche, **dadurch gekennzeichnet, dass** der Antikörper über seinen Fc-Anteil an Fc-γ-Rezeptor Typ I und/oder Typ III positive Zellen bindet.

7. Verwendung nach einem oder mehreren der vorhergehenden Ansprüche, **dadurch gekennzeichnet, dass** der bispezifische Antikörper so ausgewählt wird, dass er ein anti-CD3 X anti-tumor-assoziiertes Antigen- und/oder anti-CD4 X anti-tumor-assoziiertes Antigen- und/oder anti-CD5 X anti-tumor-assoziiertes Antigen- und/oder anti-CD6 X anti-tumor-assoziiertes Antigen- und/oder anti-CD8 X anti-tumor-assoziiertes Antigen- und/oder anti-CD2 X anti-tumor-assoziiertes Antigen- und/oder anti-CD28 X anti-tumor-assoziiertes Antigen- und/oder anti-CD40L X anti-tumor-assoziiertes Antigen- und/oder anti-CD44 X anti-tumor-assoziiertes Antigen-Antikörper ist.

8. Verwendung nach einem oder mehreren der vorhergehenden Ansprüche, **dadurch gekennzeichnet, dass** der bispezifische Antikörper aus einem heterologen bispezifischen Antikörper, bevorzugt einem heterologen Ratte/Maus bispezifischen Antikörper, ausgewählt wird.

9. Verwendung nach einem oder mehreren der vorhergehenden Ansprüche, **dadurch gekennzeichnet, dass** ein Antikörper mit einer der nachfolgenden Isotyp-Kombinationen im Fc-Bereich ausgewählt wird:
Ratte-IgG2b/Maus-IgG2a,
Ratte-IgG2b/Maus-IgG2b,
Ratte-IgG2b/human-IgG1,
Ratte-IgG2b/human-IgG2.

10. Verwendung nach einem oder mehreren der vorhergehenden Ansprüche, **dadurch gekennzeichnet, dass** der Antikörper die nachfolgende Isotypkombination besitzt:
Maus-[VH-CH1, VL-CL]-human IgG1-[hinge]- human-IgG3 [Caucasian allotypes G3m(b+g)= no binding to protein A)-[CH2-CH3] /Ratte-[VH-CH1, VL-CL]-human IgG1-[hinge]- human IgG1-[CH2-CH3];
und/oder der Antikörper ein anti-Her2/neu x anti-CD3 Antikörper oder ein anti-EpCAM x anti-CD3-Antikörper ist, der an Fc-γ-Typ I/III-Rezeptoren bindet, jeweils bevorzugt mit der Isotypkombination Ratte-IgG2b/Maus-IgG2a oder der
.Isotypkombination Maus-[VH-CH1, VL-CL]-human IgG1-[hinge]- human-IgG3 [Caucasian allotypes G3m(b+g)= no binding to protein A)-[CH2-CH3] /Ratte-[VH-CH1, VL-CL]-human IgG1-[hinge]- human IgG1-[CH2-CH3].

11. Verwendung nach einem oder mehreren der vorhergehenden Ansprüche, **dadurch gekennzeichnet, dass** der trifunktionelle, bispezifische oder trispezifische Antikörper postoperativ in zumindest einer weiteren Dosis verabreicht wird.

12. Verwendung nach Anspruch 10, **dadurch gekennzeichnet, dass** der Antikörper postoperativ in Form einer zweiten Dosis zur Aktivierung und Proliferation der Immunzellen und vorzugsweise zumindest einer dritten Dosis zur Zerstörung residueller und disseminierer Tumorzellen verabreicht wird.

13. Verwendung nach Anspruch 10 oder 11, **dadurch gekennzeichnet, dass** der Antikörper postoperativ als zweite Dosis in einer Menge von 1-20 µg, bevorzugt 5-10 µg, verabreicht wird, eine nachfolgende Dosis bevorzugt in einer Menge von 20-100 µg, bevorzugt 30-60 µg, und jede weitere nachfolgende Verabreichung bevorzugt in einer Menge von 100-500 µg, bevorzugt 100-300 µg, erfolgt.

14. Verwendung nach Anspruch 10, **dadurch gekennzeichnet, dass** der Antikörper postoperativ in einer Menge von 5 - 1000 µg, bevorzugt 10 - 500 µg, weiterhin bevorzugt 10 - 150 µg verabreicht wird, bevorzugt intraperitoneal, und weiterhin bevorzugt die postoperative Verabreichung 2 - 5 mal erfolgt.

15. Verwendung nach einem oder mehreren der vorhergehenden Ansprüche, **dadurch gekennzeichnet, dass** die Tumorzellen durch einen chirurgischen Eingriff disseminiert wurden oder dass die Tumorzellen ohne Anwendung eines chirurgischen Eingriffs vom Tumor disseminiert wurden.
